(19) Europäisches Patentamt
European Patent Office
Office européen des brevets

(11) Veröffentlichungsnummer : 0 123 727
B1

(12) # EUROPÄISCHE PATENTSCHRIFT

(45) Veröffentlichungstag der Patentschrift : 20.08.86

(51) Int. Cl.⁴ : C 07 C 153/023, A 01 N 47/30

(21) Anmeldenummer : 83112135.5

(22) Anmeldetag : 02.12.83

(54) **Substituierte Harnstoffe, Verfahren zu ihrer Herstellung und ihre Verwendung zur Bekämpfung unerwünschten Pflanzenwuchses.**

(30) Priorität : 10.12.82 DE 3245679

(43) Veröffentlichungstag der Anmeldung : 07.11.84 Patentblatt 84/45

(45) Bekanntmachung des Hinweises auf die Patenterteilung : 20.08.86 Patentblatt 86/34

(84) Benannte Vertragsstaaten : BE CH DE FR GB IT LI NL

(56) Entgegenhaltungen :
FR-A- 2 264 804
FR-A- 2 271 201
Die Akte enthält technische Angaben, die nach dem Eingang der Anmeldung eingereicht wurden und die nicht in dieser Patentschrift enthalten sind.

(73) Patentinhaber : BASF Aktiengesellschaft
Carl-Bosch-Strasse 38
D-6700 Ludwigshafen (DE)

(72) Erfinder : Lange, Arno, Dr.
Oberes Geistal 3 b
D-6702 Bad Duerkheim (DE)
Erfinder : Wuerzer, Bruno, Dr., Dipl.-Landwirt
Ruedigerstrasse 13
D-6701 Otterstadt (DE)

EP 0 123 727 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Beschreibung

Die vorliegende Erfindung betrifft substituierte Harnstoffe, Verfahren zu ihrer Herstellung, Herbizide, welche diese Verbindungen enthalten, sowie Verfahren zur Bekämpfung unerwünschten Pflanzenwuchses mit diesen Verbindungen.

Es ist bekannt, daß Alkoxycarbonylphenylharnstoffe herbizid wirksam sind (DE-OS 2 413 258 ; DE-OS 2 445 529).

Es wurde gefunden, daß substituierte Harnstoffe der Formel

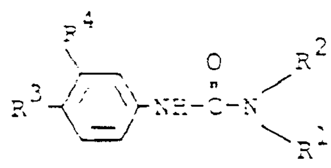

(I)

in der

$R^1$ Wasserstoff oder $C_1$-$C_4$-Alkyl,

$R^2$ Wasserstoff, $C_1$-$C_4$-Alkyl, $C_1$-$C_4$-Alkoxy, $C_2$-$C_4$-Alkenyl, $C_2$-$C_4$-Alkinyl, $C_3$-$C_7$-Cycloalkyl, durch $C_1$-$C_4$-Alkyl substituiertes $C_3$-$C_7$-Cycloalkyl, Phenyl oder durch Halogen oder $C_1$-$C_4$-Halogenalkyl substituiertes Phenyl,

$R^3$ Wasserstoff, Halogen oder den Rest —CO—S—R, wobei R für $C_1$-$C_{10}$-Alkyl, $C_3$-$C_8$-Cycloalkyl, $C_2$-$C_4$-Alkenyl, $C_2$-$C_4$-Alkinyl oder $C_7$-$C_{12}$-Aralkyl steht, und

$R^4$ Wasserstoff, Halogen oder den Rest —CO—S—R, wobei R für $C_1$-$C_{10}$-Alkyl, $C_3$-$C_8$-Cycloalkyl, $C_2$-$C_4$-Alkenyl, $C_2$-$C_4$-Alkinyl oder $C_7$-$C_{12}$-Aralkyl steht, bedeuten,

mit der Maßgabe, daß mindestens einer der Substituenten $R^3$ und $R^4$ den Rest —CO—S—R bedeutet, eine beachtliche herbizide Aktivität und eine überraschend gute Verträglichkeit gegenüber einer Reihe von Kulturpflanzen aufweisen.

$R^1$ in Formel I bedeutet Wasserstoff oder $C_1$-$C_4$-Alkyl, wie Methyl, Ethyl, n-Propyl, i-Butyl, vorzugsweise Methyl oder Ethyl. $R^2$ in Formel I bedeutet Wasserstoff, $C_1$-$C_4$-Alkyl, $C_1$-$C_4$-Alkoxy, $C_2$-$C_4$-Alkenyl, $C_2$-$C_4$-Alkinyl, gegebenenfalls durch $C_1$-$C_4$-Alkyl, vorzugsweise durch Methyl, substituiertes $C_3$-$C_7$-Cycloalkyl oder gegebenenfalls durch Halogen, vorzugsweise Chlor, oder $C_1$-$C_4$-Halogenalkyl substituiertes Phenyl, beispielsweise Methyl, Ethyl, n-Propyl, Isopropyl, tert.-Butyl, Methoxy, Ethoxy, Vinyl, Propen-1-yl-4, Ethinyl, Propin-1-yl, Propargyl, Butin-2-yl-1, Butin-1-yl-3, Butin-1-yl-4, 3-Methyl-butin-1-yl-3, Cyclopropyl, Cyclobutyl, Cyclopentyl, Cyclohexyl, 2-Methyl-cyclohexyl, 3,4-Dichlor-phenyl oder 3-Trifluormethyl-phenyl. Bevorzugt sind Methyl und Ethyl.

R im Rest —CO—S—R, der für $R^3$ und $R^4$ in Formel I stehen kann, bedeutet $C_1$-$C_{10}$-Alkyl, vorzugsweise $C_4$-$C_8$-Alkyl, $C_3$-$C_8$-Cycloalkyl, $C_2$-$C_4$-Alkenyl, $C_2$-$C_4$-Alkinyl oder $C_7$-$C_{12}$-Aralkyl, beispielsweise Methyl, Ethyl, Propyl, i-Propyl, Butyl, i-Butyl, s-Butyl, einen Pentyl-, Hexyl- oder Heptylrest, Cyclobutyl, Cyclopentyl, Cyclohexyl, Cycloheptyl, Cyclooctyl, Allyl, But-2-inyl, Benzyl, Phenethyl.

Bevorzugte Harnstoffe der Formel I sind solche, bei denen $R^1$ $C_1$-$C_4$-Alkyl, vorzugsweise Methyl, und $R^2$ Wasserstoff, $C_1$-$C_4$-Alkyl, vorzugsweise Methyl, oder $C_1$-$C_4$-Alkoxy, vorzugsweise Methoxy, bedeuten. Außerdem sind Harnstoffe dieser Struktur mit einem $C_3$-$C_8$-Cycloalkylthiocarbonylrest oder einem $C_4$-$C_8$-Alkylrest als $R^3$ und/oder $R^4$ bevorzugt.

Man erhält die substituierten Harnstoffe der Formel I durch Umsetzung von substituierten Anilinen der Formel

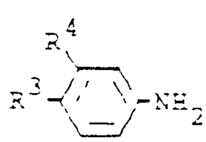

(II)

in der $R^3$ und $R^4$ die obengenannten Bedeutungen haben, mit

a) Phosgen oder Diphosgen und anschließend mit einem Amin der Formel

$$HNR^1R^2 \quad (III),$$

in der $R^1$ und $R^2$ die obengenannten Bedeutungen haben, oder

b) mit einem Carbamidsäurehalogenid der Formel

$$Hal—CO—NR^1R^2 \quad (IV),$$

in der $R^1$ und $R^2$ die obengenannten Bedeutungen haben und Hal für Halogen steht.

Weiterhin können substituierte Harnstoffe der Formel I durch Umsetzung eines Benzoylhalogenids der Formel

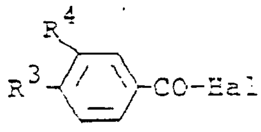

(V)

in der $R^3$ und $R^4$ die obengenannten Bedeutungen haben und Hal für Halogen steht, mit einem Alkaliazid oder Trialkylsilylazid und Überführung des so erhaltenen Säureazids in das entsprechende Isocyanat und Umsetzung des letzteren mit einem Amin der Formel III erhalten werden.

Substituierte Harnstoffe der Formel I, bei denen $R^1$ $C_1$-$C_4$-Alkyl bedeutet und $R^2$, $R^3$ und $R^4$ die obengenannten Bedeutungen haben, können auch durch Umsetzung eines substituierten Anilins der Formel II mit einem Isocyanat der Formel

$$R^1NCO \qquad (VI),$$

in der $R^1$ $C_1$-$C_4$-Alkyl bedeutet, erhalten werden.

Die oben beschriebenen Umsetzungen werden vorzugsweise in Gegenwart geeigneter Lösungs- oder Verdünnungsmittel durchgeführt. Als solche kommen praktisch alle inerten organischen Solventien in Betracht.

Hierzu gehören insbesondere aromatische und aliphatische Kohlenwasserstoffe, wie Ligroin, Benzin, Toluol, Pentan, Hexan, Cyclohexan, Petrolether, aromatische und aliphatische Halogenkohlenwasserstoffe, wie Methylenchlorid, Chloroform, Tetrachlorkohlenstoff, 1,1- und 1,2-Dichlorethan, 1,1,1- und 1,1,2-Trichlorethan, Chlorbenzol, o-, m-, p-Dichlorbenzol, o-, m-, p-Chlortoluol, aromatische und alphatische Nitrokohlenwasserstoffe, wie Nitrobenzol, Nitroethan, o-, m-, p-Nitrotoluol, Nitrile, wie Acetonitril, Butyronitril, Isobutyronitril, Ether, wie Diethylether, Di-n-propylether, Tetrahydrofuran, Dioxan, Ester, wie Acetessigester, Ethylacetat, Isobutylacetat, ferner Ketone, wie Aceton, Methylethyl-, Methylisopropyl- und Methylisobutylketon, außerdem Amide, wie Methylformamid und Dimethylformamid. Auch Gemisch dieser Lösungsmittel können verwendet werden.

Die Umsetzung der Aniline der Formel II mit den Aminen der Formel III läßt sich sehr gut durchführen, indem man das substituierte Anilin der Formel II zunächst mit einem Überschuß Phosgen in das entsprechende Isocyanat überführt (siehe Houben-Weyl, Bd. 8, S. 122-123, 1952 ; Methodicum Chimicum, Bd. 6, 1974, S. 780) und anschließend mit mindestens der äquivalenten Menge Amin der Formel III gegebenenfalls in Gegenwart eines Lösungsmittels bei einer Temperatur von —10 °C bis + 150 °C, vorzugsweise von 20 °C bis 120 °C, kontinuierlich oder diskontinuierlich umsetzt.

Zur Durchführung der Reaktion des substituierten Anilins der Formel II mit dem Carbamidsäurehalogenid der Formel IV wird das Carbamidsäurehalogenid in einem Überschuß von bis zu 50 % (Mol% oder Gew.%), gegebenenfalls in Gegenwart eines Lösungsmittels und gegebenenfalls in Gegenwart eines Säurebinders, bei einer Temperatur von —10 °C bis + 150 °C, vorzugsweise von 20 °C bis 120 °C, kontinuierlich oder diskontinuierlich umgesetzt (Houben-Weyl, Methoden der organischen Chemie 8, S. 160-161, 1952).

Als Säurebinder können alle Üblichen Säurebindemittel verwendet werden. Hierzu gehören vorzugsweise Alkalihydroxide, Alkalicarbonate, Alkalialkoholate und tertiäre organische Basen. Als besonders geeignet seien im einzelnen genannt : Natriumhydroxid, Natriumhydrogencarbonat, Natriummethylat, Triethylamin, Pyridin, Trimethylamin, alpha-, beta-Picolin, Lutidin, N,N-Dimethylanilin, N,N-Dimethylcyclohexylamin, Chinolin, Tri-n-propylamin, Tri-n-butylamin und Acridin.

Zur Durchführung des Verfahrens, ausgehend von Benzoylhalogenid der Formel V, wird zunächst 0,1 Mol Benzoylhalogenid, vorzugsweise Benzoylchlorid, mit 0,1 bis 0,4 Mol Alkaliazid oder Trimethylsilylazid im Überschuß zu dem entsprechenden Phenylisocyanat umgesetzt. Dieses wird, gegebenenfalls durch Einengen des Reaktionsgemisches, als Rohprodukt isoliert und für die weiteren Umsetzungen, gegebenenfalls in Gegenwart eines Lösungsmittels, mit der mindestens äquimolaren Menge Amin der Formel III versetzt. Vorzugsweise setzt man das Benzoesäurehalogenid der Formel V mit Trimethylsilylazid um, überführt anschließend thermisch in das entsprechende Isocyanat (Synthesis 1972, S. 551-553) und setzt danach mit einem Amin gegebenenfalls in Gegenwart eines. Lösungsmittels bei einer Temperatur von —10 °C bis + 150 °C, vorzugsweise von 20 °C bis 120 °C, kontinuierlich oder diskontinuierlich um.

Bei der Verfahrensvariante, die auf der Umsetzung von Anilin mit Isocyanat beruht, wird das substituierte Anilin der Formel II mit einem Überschuß von bis zu 50 % (Mol% oder Gew.%) Isocyanat der Formel VI, gegebenenfalls in Gegenwart eines Lösungsmittels bei einer Temperatur von —10 °C bis + 150 °C, vorzugsweise 20 °C bis 120 °C, kontinuierlich oder diskontinuierlich umgesetzt (Houben-Weyl, Methoden der org. Chemie, Bd. 8, S. 157-159, 1952).

Die Isolierung der Endprodukte erfolgt bei allen Verfahrensvarianten entweder bei aus dem Reaktionsgemisch ausgefallenen Stoffen durch Absaugen und anschließende Reinigung durch Umkristallisieren bzw. Chromatographieren oder durch Einengen des Reaktionsgemisches unter vermindertem Druck zur Trockne und Reinigung des Rückstandes durch Umkristallisieren bzw. Chromatographieren.

Die als Ausgangsstoffe verwendbaren substituierten Aniline der Formel II sind neu. Sie können durch Reduktion von Nitrobenzoesäurethiolestern der Formel

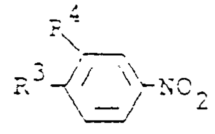

(VII)

in der $R^3$ und $R^4$ die obengenannten Bedeutungen haben, durch katalytische Hydrierung in Gegenwart eines Metalls der 8. Nebengruppe als Katalysator und in Gegenwart eines Lösungsmittels erhalten werden. Dieser Reaktionsablauf ist überraschend, da die Literatur lehrt, daß die Reduktion von Benzoesäurethiolestern mit Raney-Nickel und Wasserstoff zur Spaltung führt, so daß Benzylalkohole und Mercaptane entstehen. (Houben-Weyl, Methoden der organischen Chemie, Bd. 8, Seiten 644-645, Georg-Thieme-Verlag, Stuttgart, 4. Auflage, 1952).

Die katalytische Hydrierung wird unter an sich üblichen Bedingungen durchgeführt. Als Katalysatoren kommen alle Metalle der 8. Nebengruppe in fein verteilter Form oder auf einem Träger oder als Verbindung in Betracht. Auch Gemische können verwendet werden. Bevorzugte Katalysatoren sind Nickel, Platin und Palladium.

Als Lösungsmittel kommen alle interten Lösungsmittel in Betracht, insbesondere Alkohole, wie Methanol, Ethanol oder Propanol, Ether, wie Tetrahydrofuran, Ketone oder Ester.

Die Reaktionstemperatur kann zwischen 0 und 200 °C, vorzugsweise zwischen Raumtemperatur und 80 °C, schwanken. Die Reaktionszeit liegt zwischen 2 Stunden und 2 Tagen, vorzugsweise zwischen 6 und 36 Stunden.

Die Nitrobenzoesäurethiolester der Formel VII können nach üblichen Methoden hergestellt werden (Houben-Weyl, Methoden der organischen Chemie, Bd. 9, Seiten 754-755, Georg-Thieme-Verlag, Stuttgart, 4. Auflage, 1955).

## Beispiel 1

a) Bei Raumtemperatur werden 20.8 g 1.2-Dimethyl-n-propylmercaptan, 50 ml Ethylenchlorid und 22,2 g Triethylamin vorgelegt. Bei 10-20 °C (Eiswasserkühlung) werden 48.4 g 2-Chlor-4-nitrobenzoylchlorid zugetropft. Man hält 2 Stunden bei ca. 15 °C und rührt über Nacht bei Raumtemperatur nach. Es wird abgesaugt, das Filtrat mit Wasser, verdünnter Salzsäure, verdünnter $NaHCO_3$-Lösung und Wasser gewaschen, getrocknet und eingeengt. Man erhält 49,3 g 2-Chlor-4-nitro-benzoesäure-(1,2-dimethyl-n-propylmercapto)-ester vom $n_D^{25}$ 1,567 8.

b) 40 g 2-Chlor-4-nitro-benzoesäure-(1,2-dimethyl-n-propylmercapto)-ester werden in 150 ml Tetrahydrofuran mit 8 g Raney-Nickel bei 200 bar $H_2$-Druck und 50 °C 15 Stunden hydriert. Der Katalysator wird abgetrennt und dann eingeengt. Man erhält 33,3 g 2-Chlor-4-amino-benzoesäure-(1,2-dimethyl-n-propylmercapto)-ester vom Fp. 43-46 °C.

c) 8,24 g 2-Chlor-4-amino-benzoesäure-(1,2-dimethyl-n-propylmercapto)-ester werden ihn 80 ml Toluol vorgelegt. Man tropft insgesamt 3 g Methylisocyanat in Toluol zu und hält 2 Stunden bei 40 °C. Durch Einengen werden 10 g Rohprodukt erhalten, das ca. 80 %ig ist. Reinigung durch Säulenchromatographie (Kieselgel mit Toluol und Toluol-Aceton) ergeben 5 g N-[3-Chlor-4-(1,3-dimethyl-n-propyl-mercapto-carbonyl)-phenyl]-N'-methyl-harnstoff vom Fp. 89-95 °C (Verbindung Nr. 1).

## Beispiel 2

a) 34.8 g Cyclohexylmercaptan, 200 ml 1,1,1-Trichlorethan und 36,3 g Triethylmercaptan werden vorgelegt. Bei 10° bis 20 °C werden dann 72,6 g 2-Chlor-4-nitrobenzoylchlorid in 100 ml 1,1,1-Trichlorethan zugetropft. Dann hält man 2 Stunden bei 15 °C und über Nacht bei Raumtemperatur. Es wird abgesaugt, das Filtrat wird gründlich mit Wasser gewaschen, getrocknet und eingeengt. Man erhält 81.7 g 2-Chlor-4-nitro-benzoesäure-cyclohexylmercaptoester mit dem Brechungsindex $n_D^{25}$ 1,591 4.

b) 210 g 2-Chlor-4-nitro-benzoesäure-cyclohexylmercaptoester werden in 2 l Tetrahydrofuran gelöst, mit 15 g Raney-Nickel versetzt und 15 Stunden bei 50 °C und einem Wasserstoffdruck von 200 atm hydriert. Dann trennt man vom Katalysator ab und engt ein. Man erhält 179.5 g 2-Chlor-4-aminobenzoesäurecyclohexyl-mercaptoester vom Schmelzpunkt 97-100 °C.

c) 6.74 g 2-Chlor-4-amino-benzoesäure-cyclohexylmercapto-ester werden in 60 ml Toluol und 10 ml Pyridin gelöst. Dann tropft man 3.4 g N-Methyl-N-methoxycarbamidsäurechlorid zu. Aufgrund der exothermen Reaktion steigt die Temperatur auf 29 °C, dann wird 2 Stunden lang bei 40 °C gehalten. Es wird abgesaugt, gut mit Wasser und verdünnter Salzsäure gewaschen, getrocknet und eingeengt. Man

erhält 6 g N-(3-Chlor-4-cyclohexylmercaptocarbonyl-phenyl)-N'-methyl-N'-methoxy-harnstoff (Verbindung Nr. 37).

## Beispiel 3

a) Bei — 10 °C bis — 15 °C werden in eine Lösung von 90 g Phosgen in 150 ml 1,1,1-Trichlorethan, 107,8 g 2-Chlor-4-amino-benzoesäure-cyclohexyl-mercapto-ester in 350 ml 1,1,1-Trichlorethan eingetropft. Man heizt langsam auf und hält 3 Stunden unter Rückfluß. Nach dem Einengen erhält man 122,1 g 2-Chlor-4-isocyanato-benzoesäure-cyclohexyl-mercapto-ester vom Schmelzpunkt 60 bis 65 °C.

b) Zu überschüssiger, wäßriger Dimethylaminlösung werden bei Raumtemperatur 20 g 2-Chlor-4-isocyanatobenzoesäure-cyclohexylmercaptoester in 80 ml Toluol getropft. Man hält bei Raumtemperatur, rührt über Nacht nach und saugt ab. Man erhält 16,3 g N-(3-Chlor-4-cyclohexyl-mercaptocarbonyl-phenyl)-N',N'-dimethyl-harnstoff (Verbindung Nr. 35).

Analog können folgende Verbindungen der Formel I hergestellt werden :

| Verb. Nr. | $R^1$ | $R^2$ | $R^3$ | $R^4$ | Fp (°C)/$n_D$ |
|---|---|---|---|---|---|
| 1 | $CH_3$ | H | $-\overset{O}{\overset{\parallel}{C}}-S-CH(CH_3)-CH(CH_3)_2$ | Cl | 89 - 95 |
| 2 | $CH_3$ | $CH_3$ | " | Cl | 115 - 119 |
| 3 | $CH_3$ | $OCH_3$ | " | Cl | $n_D^{25}$ 1,5756 |
| 4 | $CH_3$ | H | H | $-\overset{O}{\overset{\parallel}{C}}-S-CH(CH_3)-CH(CH_3)_2$ | 71 - 73 |
| 5 | $CH_3$ | $CH_3$ | H | " | 69 - 71 |
| 6 | $CH_3$ | $OCH_3$ | H | " | $n_D^{25}$ 1,5607 |
| 7 | $CH_3$ | $CH_3$ | Cl | " | |
| 8 | $CH_3$ | H | Cl | " | |
| 9 | $CH_3$ | $OCH_3$ | Cl | " | |
| 10 | $CH_3$ | H | $-\overset{O}{\overset{\parallel}{C}}-S-C(CH_3)_2-CH_2-CH_3$ | H | 104 - 107 |
| 11 | $CH_3$ | $CH_3$ | " | H | 136 - 139 |
| 12 | $CH_3$ | $OCH_3$ | " | H | $n_D^{25}$ 1,5738 |
| 13 | $CH_3$ | H | H | $-\overset{O}{\overset{\parallel}{C}}-S-C(CH_3)_2-CH_2-CH_3$ | |
| 14 | $CH_3$ | $CH_3$ | H | " | |
| 15 | $CH_3$ | $OCH_3$ | H | $-\overset{O}{\overset{\parallel}{C}}-S-C(CH_3)_2-CH_2-CH_3$ | |
| 16 | $CH_3$ | H | $-\overset{O}{\overset{\parallel}{C}}-S-CH(CH_3)_2$ | Cl | |
| 17 | $CH_3$ | $CH_3$ | " | Cl | |
| 18 | $CH_3$ | $OCH_3$ | " | Cl | |
| 19 | $CH_3$ | H | $-\overset{O}{\overset{\parallel}{C}}-S-CH_3$ | Cl | |
| 20 | $CH_3$ | $CH_3$ | " | Cl | |
| 21 | $CH_3$ | $OCH_3$ | " | Cl | |
| 22 | $CH_3$ | H | $-\overset{O}{\overset{\parallel}{C}}-S-CH_2-CH(CH_3)-CH_2-C(CH_3)_3$ | H | |
| 23 | $CH_3$ | $CH_3$ | " | H | |
| 24 | $CH_3$ | $OCH_3$ | " | H | |

Fortsetzung

| Verb. Nr. | $R^1$ | $R^2$ | $R^3$ | $R^4$ | Fp (°C)/$n_D$ |
|---|---|---|---|---|---|
| 25 | $CH_3$ | H | $-\overset{O}{\overset{\|}{C}}-S-CH_2-C(CH_3)=CH_2$ | Cl | |
| 26 | $CH_3$ | $CH_3$ | " | Cl | $n_D^{25}$ 1,5945 |
| 27 | $CH_3$ | $OCH_3$ | " | Cl | $n_D^{25}$ 1,6082 |
| 28 | $CH_3$ | H | Cl | $-\overset{O}{\overset{\|}{C}}-S-CH_2-CH=CH_2$ | |
| 29 | $CH_3$ | $CH_3$ | Cl | " | |
| 30 | $CH_3$ | $OCH_3$ | Cl | " | |
| 31 | $CH_3$ | H | $-\overset{O}{\overset{\|}{C}}-S-CH_2-C(CH_3)_3$ | Cl | |
| 32 | $CH_3$ | $CH_3$ | " | Cl | |
| 33 | $CH_3$ | $OCH_3$ | " | Cl | |
| 34 | $CH_3$ | H | 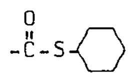 | Cl | 143 - 147 |
| 35 | $CH_3$ | $CH_3$ | " | Cl | 178 - 181 |
| 36 | $C_2H_5$ | $C_2H_5$ | " | Cl | 178 - 180 |
| 37 | $CH_3$ | $CH_3O$ | " | Cl | 94 - 96 |
| 38 | $CH_3$ | H | 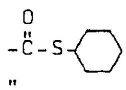 | H | 160 - 165 |
| 39 | $CH_3$ | $CH_3$ | " | H | |
| 40 | $C_2H_5$ | $C_2H_5$ | " | H | |
| 41 | $CH_3$ | $CH_3O$ | " | H | 139 - 141 |
| 42 | $CH_3$ | H | Cl | 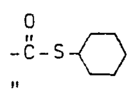 | 156-160 |
| 43 | $CH_3$ | $CH_3$ | Cl | " | 150 - 154 |
| 44 | $CH_3$ | $CH_3O$ | Cl | " | 101 - 110 |
| 45 | $CH_3$ | H | H | 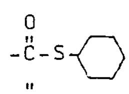 | 152 - 155 |
| 46 | $CH_3$ | $CH_3$ | H | " | |
| 47 | $CH_3$ | $CH_3O$ | H | 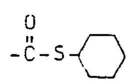 | $n_D^{25}$ 1,5719 |
| 48 | $CH_3$ | H | 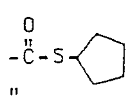 | Cl | |
| 49 | $CH_3$ | $CH_3$ | " | Cl | |
| 50 | $CH_3$ | $CH_3O$ | " | Cl | |
| 51 | $CH_3$ | H | " | H | 189 - 195 |
| 52 | $CH_3$ | $CH_3$ | " | H | |
| 53 | $CH_3$ | $CH_3O$ | " | H | 89 - 99 |
| 54 | $CH_3$ | H | Cl | 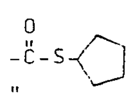 | |
| 55 | $CH_3$ | $CH_3$ | Cl | " | |
| 56 | $CH_3$ | $CH_3O$ | Cl | " | |
| 57 | $CH_3$ | H | $-\overset{O}{\overset{\|}{C}}-S-CH_2-C_6H_5$ | H | 174 - 178 |
| 58 | $CH_3$ | $CH_3$ | " | H | |
| 59 | $CH_3$ | $CH_3O$ | " | H | 53 - 65 |

Fortsetzung

| Verb. Nr. | $R^1$ | $R^2$ | $R^3$ | $R^4$ | Fp (°C)/$n_D$ |
|---|---|---|---|---|---|
| 60 | $CH_3$ | H | $-\overset{O}{\overset{\|}{C}}-S-C_2H_4-C_6H_5$ | H | |
| 61 | $CH_3$ | $CH_3$ | " | H | |
| 62 | $CH_3$ | $CH_3O$ | " | H | |
| 63 | $CH_3$ | H | $-\overset{O}{\overset{\|}{C}}-S-C_2H_4-C_6H_5$ | Cl | 143 - 147 |
| 64 | $CH_3$ | $CH_3$ | " | Cl | 131 - 134 |
| 65 | $CH_3$ | $CH_3O$ | " | Cl | $n_D^{25}$ 1,611 |
| 66 | $CH_3$ | H | H | $-\overset{O}{\overset{\|}{C}}-S-C_2H_4-C_6H_5$ | 145-149 |
| 67 | $CH_3$ | $CH_3$ | H | " | 150-152 |
| 68 | $CH_3$ | $CH_3O$ | H | " | 85- 88 |
| 69 | $CH_3$ | H | $-\overset{O}{\overset{\|}{C}}-S-CH(CH_3)-CH_2-CH_3$ | $-\overset{O}{\overset{\|}{C}}-S-CH(CH_3)-CH_2CH_3$ | |
| 70 | $CH_3$ | $CH_3$ | " | " | |
| 71 | $CH_3$ | $CH_3O$ | " | " | |
| 72 | $CH_3$ | $CH(CH_3)-C\equiv CH$ | $-\underset{O}{\underset{\|}{C}}-S-$ 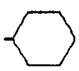 | Cl | 135 - 138 |
| 73 | H | 4-Chlorphenyl | $-\underset{O}{\underset{\|}{C}}-S-CH(CH_3)-CH(CH_3)_2$ | Cl | 152 - 154 |
| 74 | H | 2,6-Dichlor-phenyl | " | Cl | 187 - 190 |
| 75 | $CH_3$ | $CH_3$ | $-\underset{O}{\underset{\|}{C}}-S-C_2H_5$ | Cl | |
| 76 | $CH_3$ | H | H | Cl | |
| 77 | $CH_3$ | $CH_3O$ | H | Cl | |
| 78 | $CH_3$ | $CH_3$ | H | $-\underset{O}{\underset{\|}{C}}-S-C_2H_5$ | |
| 79 | $CH_3$ | H | H | " | |
| 80 | $CH_3$ | $CH_3O$ | H | " | |
| 81 | $CH_3$ | $CH_3$ | H | $-\underset{O}{\underset{\|}{C}}-S-i-C_3H_7$ | |
| 82 | $CH_3$ | H | H | " | |
| 83 | $CH_3$ | $CH_3O$ | H | " | |
| 84 | $CH_3$ | $CH_3$ | $-\overset{O}{\overset{\|}{C}}-S-CH(CH_3)C_2H_5$ | Cl | |
| 85 | $CH_3$ | H | " | Cl | |
| 86 | $CH_3$ | $CH_3O$ | " | Cl | |
| 87 | $CH_3$ | $CH_3O$ | $-\overset{O}{\overset{\|}{C}}-S-[CH(CH_3)]_2-CH_3$ | $-\overset{O}{\overset{\|}{C}}-S-[CH(CH_3)]_2-CH_3$ | $n_D^{25}$ 1,5685 |
| 88 | $CH_3$ | H | $-\overset{O}{\overset{\|}{C}}-S-[CH(CH_3)]_2-CH_3$ | $-\overset{O}{\overset{\|}{C}}-S-[CH(CH_3)]_2-CH_3$ | 182-184 |

Analog zu den in den Beispielen 1 b) und 2 b) beschriebenen Verfahren können folgende substituierte Aniline der Formel II durch Reduktion der entsprechenden Nitrobenzoesäurethiolester der Formel VII erhalten werden :

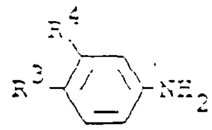

(II)

| $R^3$ | $R^4$ | Reduktionszeit [h] | Ausbeute [%] | Fp [°C]/ $n_D$ |
|---|---|---|---|---|
| $-CH(CH_3)-C(CH_3)_2$ | Cl | 15 | 92 | 43-46 |
| $-C(CH_3)_2-C_2H_5$ | H | 15 | 91 | 57-60 |
| Cyclohexyl | Cl | 15 | 95 | 77-81 |
| " | H | 15 | 91 | 85-87 |
| H | $-CH(CH_3)-C(CH_3)_2$ | 15 | 96 | $n_D^{25}$ 1,5860 |
| $-CH_2-C_6H_5$ | H | 15 | 74 | 81-105 |
| H | Cyclohexyl | 15 | 93 | 38-43 |
| Cl | " | 15 | 90 | 74-77 |
| $-C_2H_4-C_6H_5$ | Cl | 15 | 94 | 143-146 |
| Cyclopentyl | Cl | 30 | 77 | 101-103 |
| Cl | Cyclopentyl | 15 | 91 | 69-72 |
| Cyclopentyl | H | 60 | 86 | 80-95 |

Die substituierten Harnstoffe der Formel I können beispielsweise in Form von direkt versprühbaren Lösungen. Pulvern. Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B.

Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind :

I. Man vermischt 90 Gewichtsteile des Wirkstoffs Nr. 1 mit 10 Gewichtsteilen N-Methyl-alphapyrrolidon und erhält eine Lösung, die zur Anwendung in Form kleinster Tropfen geeignet ist.

II. 10 Gewichtsteile des Wirkstoffs Nr. 6 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-monoethanolamid, 2 Gewichtsteilen Calciumsalz der Dodecyl-benzolsulfonsäure und 2 Gewichtsteilen des Anlagerungsproduktes von 40 Mol Ethylenoxid an 1 Mol Ricinusöl besteht.

III. 20 Gewichtsteile des Wirkstoffs Nr. 34 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

IV. 20 Gewichtsteile des Wirkstoffs Nr. 37 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 80 Gewichtsteile des Wirkstoffs Nr. 3 werden mit 3 Gewichtsteilen des Natriumsalzes der Diisobutyl-naphthalin-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammernühle vermahlen.

VI. 5 Gewichtsteile des Wirkstoffs Nr. 34 werden mit 95 Gewichtsteilen feinteilingem Kaolin vermischt. Man erhält auf diese Weise ein Stäubemittel, das 5 Gewichtsprozent des Wirkstoffs enthält.

VII. 30 Gewichtsteile des Wirkstoffs Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile des Wirkstoffs Nr. 2 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadien 0,05 bis 5, vorzugsweise 0,1 bis 1,0 kg/ha.

Die herbizide Wirkung der substituierten Harnstoffderivate der Formel I auf das Wachstum von unerwünschten und erwünschten Pflanzen wird durch folgende Gewächshausversuche gezeigt :

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmenge beträgt 3,0 kg Wirkstoff/ha. Nach dem Aufbringen der Mittel werden die Gefäße leicht beregnet, um Keimung und Wachstum in Gang zu bringen. Danach deckt man die

Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Es werden direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung variieren je nach Wirkstoff ; sie betragen beispielsweise zwischen 0,125 und 1,0 kg Wirkstoff ha. Eine Abdeckung unterbleibt bei der Nachauflaufbehandlung.

Die Versuchsgefäße werden im Gewächshaus aufgestellt, wobei für wärmeliebende Arten wärmere Bereicne (20 bis 35 °C) und für solche gemäßigter Klimate 10 bis 20 °C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen :

Amaranthus spp. (zurückgekrümmte Fuchsschwanzarten), Arachys hypogae (Erdnüsse), Cassia tora, Chenopodium album (weißer Gänsefuß), Euphorbia heterophylla (Wolfsmilchart), Galium aparine (Klettenlabkraut), Glycine max (Sojabohnen), Ipomoea spp. (Prunkwindearten), Lamium amplexicaule (stengelumfassende Taubnessel), Mercurialis annua (einjähriges Bingelkraut), Oryza sativa (Reis), Sesbania exaltata (Turibaum), Sinapis alba (weißer Senf), Solanum nigrum (schwarzer Nachtschatten), Triticum aestivum (Weizen), Zea mays (Mais).

Bei Vorauflaufanwendung von 3,0 kg Wirkstroff/ha zeigen beispielsweise die Verbindungen Nr. 34 und 37 eine gute herbizide Aktivität.

Bei Nachauflaufanwendung von 0,125 kg Wirkstoff/ha bekämpfen beispielsweise die Verbindungen Nr. 6 und Nr. 37 breitblättrige unerwünschte Pflanzen ; Reis und Weizen werden nicht geschädigt. Verbindung Nr. 1 ist bei einer Aufwandmenge von 0,25 kg/ha selektiv für Soja, Verbindung Nr. 2 ist bei gleicher Aufwandmenge selektiv für Mais. Mit der gleichen Aufwandmenge bekämpfen beispielsweise die Verbindungen Nr. 34, 35, 65 und 72 breitblättrige Unkräuter selektiv in Erdnüssen und Weizen, Verbindung Nr. 44 ist selektiv wirksam gegen breitblättrige Unkräuter in Weizen und Mais. Die Verbindungen Nr. 3 und 4 bekämpfen beispielsweise mit 0,5 kg/ha breitblättrige Unkräuter in Erdnüssen.

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen :

| Botanischer Name | Deutscher Name |
|---|---|
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor — Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |

Fortsetzung

| Botanischer Name | Deutscher Name |
|---|---|
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Metha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus mungo | Erdbohne |
| Phaseolus vulgaris | Buschbohnen |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus duscis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sasamum indicum | Sesam |
| Solanum tuberosum | Kartoffel |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (v. unguiculata) | Kuhbohne |
| Vitis vinifera | Weinrebe |

Zur Verbreiterung des Wirkungsspektrums und zur Erzielung synergistischer Effekte können die substituierten Harnstoffe der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate und andere in Betracht.

Außerdem kann es von Nutzen sein, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

**Patentansprüche**

1. Substituierte Harnstoffe der Formel I

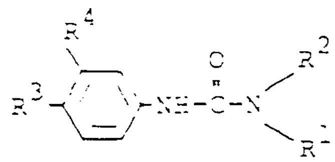

(I)

in der

$R^1$ Wasserstoff oder $C_1$-$C_4$-Alkyl,

$R^2$ Wasserstoff, $C_1$-$C_4$-Alkyl, $C_1$-$C_4$-Alkoxy, $C_2$-$C_4$-Alkenyl, $C_2$-$C_4$-Alkinyl, $C_3$-$C_7$-Cycloalkyl, durch $C_1$-$C_4$-Alkyl substituiertes $C_3$-$C_7$-Cycloalkyl, Phenyl oder durch Halogen oder $C_1$-$C_4$-Halogenalkyl substituiertes Phenyl,

$R^3$ Wasserstoff, Halogen oder den Rest —CO—S—R, wobei R für $C_1$-$C_{10}$-Alkyl, $C_3$-$C_8$-Cycloalkyl, $C_2$-$C_4$-Alkenyl, $C_2$-$C_4$-Alkinyl oder $C_7$-$C_{12}$-Aralkyl steht, und

$R^4$ Wasserstoff, Halogen oder den Rest —CO—S—R, wobei R für $C_1$-$C_{10}$-Alkyl, $C_3$-$C_8$-Cycloalkyl, $C_2$-$C_4$-Alkenyl, $C_2$-$C_4$-Alkinyl oder $C_7$-$C_{12}$-Aralkyl steht, bedeuten,

mit der Maßgabe, daß mindestens einer der Substituenten $R^3$ und $R^4$ den Rest —CO—S—R bedeutet.

2. Substituierte Harnstoffe der Formel I gemäß Anspruch 1, dadurch gekennzeichnet, daß $R^1$ $C_1$-$C_4$-Alkyl und $R^2$ Wasserstoff, $C_1$-$C_4$-Alkyl oder $C_1$-$C_4$-Alkoxy bedeuten.

3. Substituierte Harnstoffe der Formel I gemäß Anspruch 1, dadurch gekennzeichnet, daß $R^1$ $C_1$-$C_4$-Alkyl, $R^2$ $C_1$-$C_4$-Alkyl oder $C_1$-$C_4$-Alkoxy und $R^3$ $C_3$-$C_8$-Cycloalkylthiocarbonyl oder $C_4$-$C_8$-Alkylthiocarbonyl bedeuten.

4. Substituierte Harnstoffe der Formel I gemäß Anspruch 1, dadurch gekennzeichnet, daß $R^1$ $C_1$-$C_4$-Alkyl, $R^2$ $C_1$-$C_4$-Alkyl oder $C_1$-$C_4$-Alkoxy und $R^4$ $C_3$-$C_8$-Cycloalkylthiocarbonyl oder $C_4$-$C_8$-Alkylthiocarbonyl bedeuten.

5. Verfahren zur Herstellung von substituierten Harnstoffen der Formel I gemäß Anspruch 1, dadurch gekennzeichnet, daß man ein substituiertes Anilin der Formel

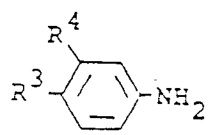

(II)

in der $R^3$ und $R^4$ die im Anspruch 1 genannten Bedeutungen haben,

a) mit Phosgen oder Diphosgen und anschließend mit einem Amin der Formel

$$HNR^1R^2 \qquad (III),$$

in der $R^2$ und $R^1$ die im Anspruch 1 genannten Bedeutungen haben, oder

b) mit einem mit einem Carbamidsäurehalogenid der Formel

$$Hal—CO—NR^1R^2 \qquad (IV),$$

in der $R^1$ und $R^2$ die im Anspruch 1 genannten Bedeutungen haben und Hal für Halogen steht, umsetzt.

6. Verfahren zur Herstellung von substituierten Harnstoffen der Formel I gemäß Anspruch 1, dadurch gekennzeichnet, daß man ein Benzoylhalogenid der Formel

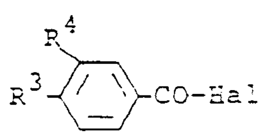

(V)

in der $R^3$ und $R^4$ die im Anspruch 1 genannten Bedeutungen haben und Hal für Halogen steht, mit einem Alkaliazid oder Trialkylsilylazid umsetzt und das so erhaltene Säureazid in das entsprechende Isocyanat überführt und letzteres mit einem Amin der Formel III gemäß Anspruch 5 umsetzt.

7. Verfahren zur Herstellung von substituierten Harnstoffen der Formel I gemäß Anspruch 1, wobei $R^1$ $C_1$-$C_4$-Alkyl bedeutet und $R^2$, $R^3$ und $R^4$ die im Anspruch 1 genannten Bedeutungen haben, dadurch gekennzeichnet, daß man ein substituiertes Anilin der Formel II gemäß Anspruch 5 mit einem Isocyanat der Formel

$$R^1NCO \qquad (VI),$$

in der $R^1$ $C_1$-$C_4$-Alkyl bedeutet, in an sich üblicher Weise umsetzt.

8. Substituierte Aniline der Formel

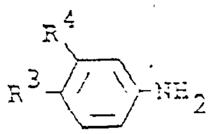

(II)

in der

$R^3$ Wasserstoff, Halogen oder den Rest —CO—S—R, wobei R für $C_1$-$C_{10}$-Alkyl, $C_3$-$C_8$-Cycloalkyl, $C_2$-$C_4$-Alkenyl, $C_2$-$C_4$-Alkinyl oder $C_7$-$C_{12}$-Aralkyl steht, und

$R^4$ Wasserstoff, Halogen oder den Rest —CO—S—R, wobei R für $C_1$-$C_{10}$-Alkyl, $C_3$-$C_8$-Cycloalkyl, $C_2$-$C_4$-Alkenyl, $C_2$-$C_4$-Alkinyl, $C_7$-$C_{12}$-Aralkyl steht, bedeuten,

mit der Maßgabe, daß mindestens einer der Substituenten $R^3$ und $R^4$ den Rest —CO—S—R bedeutet.

9. Herbizid, enthaltend einen substituierten Harnstoff der Formel I gemäß Anspruch 1.

10. Herbizid, enthaltend inerte Zusatzstoffe und einen substituierten Harnstoff der Formel I gemäß Anspruch 1.

11. Herbizid, enthaltend inerte Zusatzstoffe und einen substituierten Harnstoff der Formel I gemäß Anspruch 2.

12. Herbizid, enthaltend inerte Zusatzstoffe und einen substituierten Harnstoff der Formel I gemäß Anspruch 3.

13. Herbizid, enthaltend inerte Zusatzstoffe und einen substituierten Harnstoff der Formel I gemäß Anspruch 4.

14. Verfahren zur Bekämpfung von unerwünschtem Pflanzenwuchs, dadurch gekennzeichnet, daß man die unerwünschten Pflanzen oder von unerwünschtem Pflanzenwuchs freizuhaltende Flächen mit einer herbizid wirksamen Menge eines substituierten Harnstoffs der Formel I gemäß Anspruch 1 behandelt.

## Claims

1. A substituted urea of the formula I

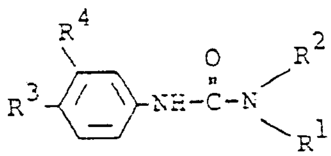

(I)

where

$R^1$ is hydrogen or $C_1$-$C_4$-alkyl,

$R^2$ is hydrogen, $C_1$-$C_4$-alkyl, $C_1$-$C_4$-alkoxy, $C_2$-$C_4$-alkenyl, $C_2$-$C_4$-alkynyl, unsubstituted or $C_1$-$C_4$-alkyl-substituted $C_3$-$C_7$-cycloalkyl, or phenyl which is unsubstituted or substituted by halogen or by $C_1$-$C_4$-haloalkyl,

$R^3$ is hydrogen, halogen or a radical —CO—S—R, where R is $C_1$-$C_{10}$-alkyl, $C_3$-$C_8$-cycloalkyl, $C_2$-$C_4$-alkenyl, $C_2$-$C_4$-alkynyl or $C_7$-$C_{12}$-aralkyl, and

$R^4$ is hydrogen, halogen or a radical —CO—S—R, where R is $C_1$-$C_{10}$-alkyl, $C_3$-$C_8$-cycloakyl, $C_2$-$C_4$-alkenyl, $C_2$-$C_4$-alkynyl or $C_7$-$C_{12}$-aralkyl,

with the proviso that at least one of the substituents $R^3$ and $R^4$ is —CO—S—R.

2. A substituted urea of the formula I as claimed in claim 1, where $R^1$ is $C_1$-$C_4$-alkyl and $R^2$ is hydrogen, $C_1$-$C_4$-alkyl or $C_1$-$C_4$-alkoxy.

3. A substituted urea of the formula I as claimed in claim 1, where $R^1$ is $C_1$-$C_4$-alkyl, $R^2$ is $C_1$-$C_4$-alkyl or $C_1$-$C_4$-alkoxy, and $R^3$ is $C_3$-$C_8$-cycloalkylthiocarbonyl or $C_4$-$C_8$-alkylthiocarbonyl.

4. A substituted urea of the formula I as claimed in claim 1, where $R^1$ is $C_1$-$C_4$-alkyl, $R^2$ is $C_1$-$C_4$-alkyl or $C_1$-$C_4$-alkoxy, and $R^4$ is $C_3$-$C_8$-cycloalkylthiocarbonyl or $C_4$-$C_8$-alkylthiocarbonyl.

5. A process for the preparation of a substituted urea of the formula I as claimed in claim 1, wherein a substituted aniline of the formula

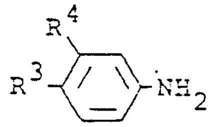

(II)

where $R^3$ and $R^4$ have the meanings given in claim 1,

a) is reacted with phosgene or diphosgene, and the product is then reacted with an amine of the formula

$$HNR^1R^2 \qquad (III),$$

where $R^1$ and $R^2$ have the meanings given in claim 1, or

b) is reacted with a carbamyl halide of the formula

$$Hal—CO—NR^1R^2 \quad (IV),$$

where $R^1$ and $R^2$ have the meanings given in claim 1, and Hal is halogen.

6. A process for the preparation of a substituted urea of the formula I as claimed in claim 1, wherein a benzoyl halide of the formula

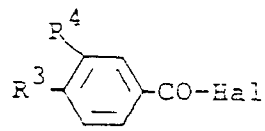

(V)

where $R^3$ and $R^4$ have the meanings given in claim 1, and Hal is halogen, is reacted with an alkali metal azide or a trialkylsilyl azide, the resulting acid azide is converted into the corresponding isocyanate and the latter is reacted with an amine of the formula III as claimed in claim 5.

7. A process for the preparation of a substituted urea of the formula I as claimed in claim 1, $R^1$ being $C_1$-$C_4$-alkyl and $R^2$, $R^3$ and $R^4$ having the meanings given in claim 1, wherein a substituted aniline of the formula II as claimed in claim 5 is reacted in a conventional manner with an isocyanate of the formula

$$R^1NCO \quad (VI),$$

where $R^1$ is $C_1$-$C_4$-alkyl.

8. A substituted aniline of the formula

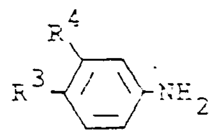

(II)

where

$R^3$ is hydrogen, halogen or a radical —CO—S—R, where R is $C_1$-$C_{10}$-alkyl, $C_3$-$C_8$-cycloalkyl, $C_2$-$C_4$-alkenyl, $C_2$-$C_4$-alkynyl or $C_7$-$C_{12}$-aralkyl, and

$R^4$ is hydrogen, halogen or a radical —CO—S—R, where R is $C_1$-$C_{10}$-alkyl, $C_3$-$C_8$-cycloalkyl, $C_2$-$C_4$-alkenyl, $C_2$-$C_4$-alkynyl or $C_7$-$C_{12}$-aralkyl,

with the proviso that at least one of the substituents $R^3$ and $R^4$ is —CO—S—R.

9. A herbicide containing a substituted urea of the formula I as claimed in claim 1.

10. A herbicide containing inert additives and a substituted urea of the formula I as claimed in claim 1.

11. A herbicide containing inert additives and a substituted urea of the formula I as claimed in claim 2.

12. A herbicide containing inert additives and a substituted urea of the formula I as claimed in claim 3.

13. A herbicide containing inert additives and a substituted urea of the formula I as claimed in claim 4.

14. A process for combating the growth of unwanted plants, wherein the unwanted plants or the area to be kept free from unwanted plant growth are treated with a herbicidally effective amount of a substituted urea of the formula I as claimed in claim 1.

**Revendications**

1. Urées substituées de formule I

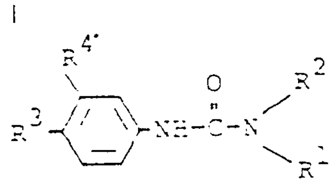

(I)

dans laquelle

$R^1$ représente hydrogène ou alkyle en $C_1$-$C_4$,

$R^2$ hydrogène, alkyle en $C_1$-$C_4$, alcoxy en $C_1$-$C_4$, alcényle en $C_2$-$C_4$, alkinyle en $C_2$-$C_4$, cycloalkyle en $C_3$-$C_7$, cycloalkyle en $C_3$-$C_7$ substitué par alkyle en $C_1$-$C_4$, phényle ou phényle substitué par halogène ou halogénalkyle en $C_1$-$C_4$.

$R^3$ hydrogène, halogène ou le reste —CO—S—R, R étant mis pour alkyle en $C_1$-$C_{10}$, cycloalkyle en $C_3$-$C_8$, alcényle en $C_2$-$C_4$, alkinyle en $C_2$-$C_4$ ou aralkyle en $C_7$-$C_{12}$ et

$R^4$ hydrogène, halogène ou le reste —CO—S—R, R étant mis pour alkyle en $C_1$-$C_{10}$, cycloalkyle en $C_3$-$C_8$, alcényle en $C_2$-$C_4$, alkinyle en $C_2$-$C_4$ ou aralkyle en $C_7$-$C_{12}$ sous réserve qu'au moins un des substituants $R^3$ et $R^4$ représente le reste —CO—S—R.

2. Urées substituées de formule I, selon la revendication 1, caractérisées par le fait que $R^1$ représente alkyle en $C_1$-$C_4$ et $R^2$ hydrogène, alkyle en $C_1$-$C_4$ ou alcoxy en $C_1$-$C_4$.

3. Urées substituées de formule I, selon la revendication 1, caractérisées par le fait que $R^1$ représente alkyle en $C_1$-$C_4$, $R^2$, alkyle en $C_1$-$C_4$ ou alcoxy en $C_1$-$C_4$ et $R^3$, cycloalkylthiocarbonyle en $C_3$-$C_8$ ou alkylthiocarbonyle en $C_4$-$C_8$.

4. Urées substituées de formule I, selon la revendication 1, caractérisées par le fait que $R^1$ représente alkyle en $C_1$-$C_4$, $R^2$, alkyle en $C_1$-$C_4$ ou alcoxy en $C_1$-$C_4$ et $R^4$, cycloalkylthiocarbonyle en $C_3$-$C_8$ ou alkylthiocarbonyle en $C_4$-$C_8$.

5. Procédé de préparation d'urées substituées de formule I selon la revendication 1, caractérisé par le fait que l'on fait réagir une aniline substituée de formule

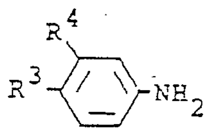 (II)

dans laquelle $R^3$ et $R^4$ ont les significations indiquées dans la revendication 1,

a) avec du phosgène ou diphosgène et ensuite avec une amine de formule

$$HNR^1R^2 \qquad (III),$$

dans laquelle $R^2$ et $R^1$ ont les significations indiquées dans la revendication 1, ou

b) avec un halogénure d'acide carbamique de formule

$$Hal—CO—NR^1R^2 \qquad (IV),$$

dans laquelle $R^1$ et $R^2$ ont les significations données dans la revendication 1 et Hal est mis pour halogène.

6. Procédé de préparation d'urées substituées de formule I selon la revendication 1, caractérisé par le fait que l'on fait réagir un halogénure de benzoyle de formule

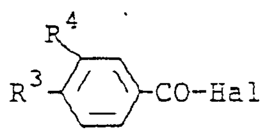 (V)

dans laquelle $R^3$ et $R^4$ ont les significations indiquées selon la revendication 1 et Hal est mis pour halogène, avec un alcaliazide ou trialkylsilylazide, et on transforme l'azide d'acide ainsi obtenu en l'isocyanate correspondant et on fait réagir ce dernier avec une amine de formule III selon la revendication 5.

7. Procédé de préparation d'urées substituées de formule I selon la revendication 1, $R^1$ représentant alkyle en $C_1$-$C_4$ et $R^2$, $R^3$ et $R^4$ ayant les significations indiquées dans la revendication 1, caractérisé par le fait qu'on fait réagir, de manière usuelle, une aniline substituée de formule II selon la revendication 5 avec un isocyanate de formule

$$R^1NCO \qquad (VI)$$

dans laquelle $R^1$ représente alkyle en $C_1$-$C_4$.

8. Anilines substituées de formule

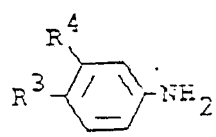 (II)

dans laquelle

$R^3$ représente hydrogène, halogène ou le reste —CO—S—R, R étant mis pour alkyle en $C_1$-$C_{10}$, cycloalkyle en $C_3$-$C_8$, alcényle ou $C_2$-$C_4$, alkinyle en $C_2$-$C_4$ ou aralkyle en $C_7$-$C_{12}$ et,

$R^4$ hydrogène, halogène ou le reste —CO—S—R, R étant mis pour alkyle en $C_1$-$C_{10}$, cycloalkyle en $C_3$-$C_8$, alcényle en $C_2$-$C_4$, alkinyle en $C_2$-$C_4$ ou aralkyle en $C_7$-$C_{12}$ sous réserve qu'au moins un des substituants $R^3$ et $R^4$ représente le reste —CO—S—R.

9. Herbicide contenant une urée substituée de formule I selon la revendication 1.

10. Herbicide contenant des additifs inertes et une urée substituée de formule I selon la revendication 1.

11. Herbicide contenant des additifs inertes et une urée substituée de formule I selon la revendication 2.

12. Herbicide contenant des additifs inertes et une urée substituée de formule I selon la revendication 3.

13. Herbicide contenant des additifs inertes et une urée substituée de formule I selon la revendication 4.

14. Procédé pour lutter contre la croissance de plantes indésirables, caractérisé par le fait que l'on traite les plantes indesirables ou les surfaces à préserver d'une croissance des plantes indésirables avec une quantité efficace, au point de vue herbicide, d'une urée substituée de formule I selon la revendication 1.